# EUROPEAN PATENT SPECIFICATION

(11) **EP 4 106 634 B1**
(45) Date of publication and mention of the grant of the patent: **24.06.2026**
(21) Application number: 21707408.7
(22) Date of filing: 19.02.2021
(51) Int. Cl.: A61B 8/08, A61B 8/12, A61B 18/00, A61M 25/10

(54) **IMPROVED SYSTEM WITH AN INFLATABLE MEMBER AND IMAGING DEVICE FOR BEING ARRANGED IN THE PATIENT'S RESPIRATORY TRACT**
VERBESSERTES SYSTEM MIT EINEM AUFBLASBAREN ELEMENT UND EINER BILDGEBENDEN VORRICHTUNG ZUR ANORDNUNG IN DEN ATEMWEGEN EINES PATIENTEN
SYSTÈME AMÉLIORÉ COMPRENANT UN ÉLÉMENT GONFLABLE ET UN DISPOSITIF D'IMAGERIE DESTINÉ À ÊTRE DISPOSÉ DANS LE TRACTUS RESPIRATOIRE D'UN PATIENT

(30) Priority: 21.02.2020 NL 2024964
(43) Date of publication of application: 28.12.2022
(73) Proprietor: Stroke2Prevent BV, 8024 AA Zwolle (NL)
(72) Inventor: NIERICH, Arno, 8051 CJ Hattem (NL)
(74) Representative: Arnold & Siedsma
(86) International application number: PCT/NL2021/050120
(87) International publication number: WO 2021/167466

(56) References cited:
- WO-A1-99/64099
- US-A- 5 331 947
- US-A1- 2019 321 001

## Description

### TECHNICAL FIELD

The field of the invention relates to systems with an inflatable member configured for being arranged in part of the patient's respiratory tract, and in particular to systems for ultrasonic imaging.

### BACKGROUND

WO 00/53098 relates to an ultrasonic imaging method that is known as transesophageal echocardiography, now referred to as TEE. This method has become a widely used imaging technique for evaluating cardiac structure, function, and valvular anatomy. TEE has also provided a new perspective on the thoracic aorta, and there is growing evidence that the technique contributes valuable and sometimes unique information about aortic structure and pathology.

TEE involves introducing an echo probe into the patient's esophagus and transmitting ultrasound waves across the thorax in the direction of the heart and aorta. However, visualization of the ascending aorta by internal TEE is limited by an air structure, i.e. the trachea and main left and right bronchi. This is due to an important physical limitation of ultrasound: absorption of ultrasound waves. This absorption is dependent of the medium and expressed in terms of the "half power distance": the distance in which half of the ultrasound energy will be absorbed. For water this is 360 cm, bone 0,2 cm and for air 0,06 cm. This means that in practice ultrasound waves will not travel through bone or air.

In order to solve this problem, WO 00/53098 proposes the use of an inflatable member that may be arranged in the trachea or in one of the bronchi and that may be filled with an ultrasonic transmission fluid, e.g. water or a saline solution in minor concentrations. This technique can only be done during operative surgery, when the patient is mechanically ventilated or on cardiopulmonary bypass, since in order to be effective the inflatable member has to completely fill and block the trachea or bronchus.

US5331947A refers to an inflatable sheath for ultrasonic imaging, with a fiber optic endoscope, of a bodily passageway or cavity area of interest. The inflatable sheath has an ultrasound transducer therein.

WO9964099A1 refers to arterial and venous cannulas or catheters incorporating means for tissue transillumination for positioning and for monitoring of the device within a blood vessel. US2019/321001A1 discloses a system with at least one inflatable member configured for being arranged in a part of the patient's respiratory tract.

### SUMMARY OF THE INVENTION

A first problem which arises when trying to introduce the inflatable member in the left bronchus, which is the position of choice when visualizing the aorta ascendens, is that the flexible catheter carrying the inflatable member is hard to manipulate. The positioning of the distal end of the flexible catheter in front of the left bronchus, so that the balloon may be lowered into that bronchus, is often a matter of trial and error. Secondly, during the arrangement of the inflatable member in the patient's respiratory tract, contact between the TEE device and the trachea wall occurs which potentially causes tissue damage. Since this positioning has to be performed during operative surgery, when timing is often critical, there is a need for an improvement.

The object of the invention is to provide a system with an inflatable member configured for being arranged in part of the patient's respiratory tract according to claim 1, with further, optional features being defined in the dependent claims.

### SUMMARY OF THE DISCLOSURE

According to a first aspect of the disclosure there is provided a system with an inflatable member configured for being arranged in part of the patient's respiratory tract. The system comprises a catheter and an imaging device, preferably a visible light imaging device. The catheter carries an inflatable member to be arranged in the respiratory tract. The catheter is provided with a fluid line for filling the inflatable member with a fluid. The catheter has a first end intended for being located outside of the patient's body and a second end intended for being located in the patient's respiratory tract. The imaging device is attached to or integrated with the second end of the catheter.

Examamples of the disclosure are based inter alia on the insight that by attaching or integrating an imaging device to the second end, the positioning of the inflatable member is done more effectively. Indeed, the operator acquires more feedback, namely the images captured by the imaging device during the procedure, and the catheter is easier to manipulate. This results in a lower chance of tissue damage.

Preferably the imaging device comprises a light source which comprises a plurality of light emitting diodes arranged along a periphery of the second end of the catheter, preferably at least three light emitting diodes equally distributed along the periphery of the second end of the catheter.

The imaging device is configured and arranged for positioning the second end of the catheter in for example the patient's respiratory tract or the patient's left main bronchus. In other words, the second end with the imaging device is shaped such that the second end forms a stylet which functions as a guide when inserting the inflatable member.

According to an example the system comprises an ultrasonic imaging device. Preferably this ultrasonic imaging device is configured to be arranged into the patient's esophagus and is configured to transmit ultrasound waves, across the thorax, through the inflatable member and to receive reflected ultrasound waves. This system allows the evaluation of an organ in a patient's body by ultrasound.

According to an example the imaging device is configured to transmit the data gathered to a device outside the patient's body so it is accessible to an operator, for example a surgeon or an assistant during a medical procedure. This transmission is done in a wired or wireless manner. In a preferred embodiment with wired transmission, a wire is arranged inside the catheter but the skilled person understands that other arrangements are possible, for example in the wall of the catheter.

According to an example the imaging device comprises a cylindrical housing aligned with a longitudinal axis of the catheter. Preferably, the cylindrical housing is arranged at least partially in the second end of the catheter, more preferably substantially entirely in the second end of the catheter. Preferably, the housing comprises a metal such as stainless steel or a plastic such as polyvinylchloride. These materials are more rigid than the flexible catheter. Since the housing is cylindrical and arranged at least partially in the second end of said catheter, the second end is given a stylet shape which can easily be inserted and can fulfil the role of guiding the catheter. The flexible catheter is easier to manipulate with the presence of the housing. Preferably, the housing has a diameter between 0.01 mm and 9 mm, more preferably 0.5 mm and 5 mm, more preferably 1 mm and 3 mm. Preferably, a length of the housing is smaller than 10 mm, more preferably smaller than 7 mm. The housing may be adhesively fixed to the second end of the catheter. In this way, the housing and the catheter cooperate to allow the inflatable member to be in an improved manner positioned.

The imaging device further comprises an image sensor, preferably an active pixel sensor such as a complementary metal oxide semiconductor (CMOS) sensor. Alternatively, the image sensor may be a charge-coupled device (CCD). Preferably, the image sensor is surrounded by the plurality of light emitting diodes, more preferably in a way so that they are equally distributed. Optionally, optical fibers may be used to provide light next to the image sensor. In this manner, the quality of the obtained image data can be improved.

According to an example the imaging device comprises a lens arranged in front of the image sensor and protrudes out of the second end of the catheter. Preferably, the lens is adhesively fixed to the image sensor and/or the housing and/or the catheter. In this way the lens forms a protection layer between the image sensor and particles that could potentially interfere with the working of the image sensor. Preferably, the image sensor has a field of view between 90 and 180 degrees oriented away from the second end of the catheter.

According to an example the system comprises a fluid line. In some embodiments, the catheter forms the fluid line. The fluid line may be formed centrally in the catheter or in the wall of the catheter. In other embodiments the fluid line is a separate line arranged inside or outside the catheter. The fluid line brings a fluid, preferably an ultrasonic fluid from an outside fluid source towards the inflatable member. In this way, air volumes that interfere with ultrasonic imaging are limited.

According to an example the system comprises at least one electrical wire arranged in the catheter and configured for connecting the imaging device with a device outside the patient's body, typically a computer with a display screen on which the image data is displayed. In other embodiments, the imaging device may be configured to communicate with a device outside the patient's body without the use of a wire. In such cases the power to operate the imaging device may come from an internal energy storage means.

According to an example the system comprises a catheter which is made from a flexible material, preferably a polymer material. These materials could be polyvinylchloride, silicone or synthetic latex. In this way the material makes the catheter flexible and pliable which allows trauma-free catheterization. The catheter may be coated with for example a polytetrafluorethylene. In this way, the catheter is better protected against acids, solvents and corrosion. On top of that, the adhesion coefficient is lower which also allows a trauma-free catheterization.

According to an example the inflatable member in non-expanded state has a volume between 20 ml and 60 ml, preferable between 30 ml and 50 ml. The material of said inflatable member is a thermoplastic elastomer, preferably a thermoplastic polyurethane elastomer.

According to yet another example the system further comprises a pressure monitoring and control member to be arranged outside the patient's body. This member is in fluid communication with the inflatable member and is configured to receive fluid from the inflatable member during operation when the pressure inside the inflatable member increases above a predetermined threshold. The control member returns the received fluid when the pressure decreases below said threshold. This pressure monitoring and control member is an elastically expandable balloon made from a material which is configured to expand elastically when the pressure in the balloon increases above the threshold pressure. When the pressure decreases below the threshold, the monitoring and control member contracts. In this way high pressure which can potentially result in tissue damage is avoided while enough pressure to limit air volumes is still maintained.

### BRIEF DESCRIPTION OF FIGURES

The accompanying drawings are used to illustrate presently preferred non-limiting example systems of the present disclosure. The above and other advantages of the features and objects of the disclosure will become more apparent and the disclosure will be better understood from the following detailed description when read in conjunction with the accompanying drawings, in which:
Figure 1 illustrates a partial sectional view of a patient's upper body showing an example of a system with an inflatable member arranged in a patient's body.
Figure 2 is a schematic view of another example of a system of the disclosure, including an imaging device, showing an inflatable member, a pressure and control member and a device outside the patient body configured and arranged to receive the data from the imaging device.
Figure 3 is an enlarged view of the second end of the catheter from figure 2 showing a more detailed schematic view of the imaging device and the inflatable member.
Figure 4A to 4C are cross sections of different examples of the secondary end of the catheter showing an imaging device.
Figure 5 is a perspective view of an example of an imaging device showing the housing, multiple electrical wires, a light source and an image sensor.
Figure 6A to 6D show examples of the imaging device with various possibilities for the positioning of the lens.
Figure 7 is a schematic view of an example of another system of the disclosure, showing the inflatable member filled with transmission fluid.
Figure 8 is a detailed, enlarged view of the encircled area VIII in figure 7.
Figure 9 is a view similar to the view of figure 8 showing yet another example of a system of the disclosure.

### DESCRIPTION OF EMBODIMENTS

For purposes of the description hereinafter, the terms "upper", "lower", "right", "left", "vertical", "horizontal", "top", "bottom", "lateral", "longitudinal" and derivatives thereof shall relate to the invention as it is oriented in the drawing figures. However, it is to be understood that the invention may assume various alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

It is to be understood that the invention may assume various alternative variations and step sequences, except where expressly specified to the contrary. It is also to be understood that the specific devices and processes illustrated in the attached drawings, and described in the following specification, are simply exemplary embodiments of the invention.

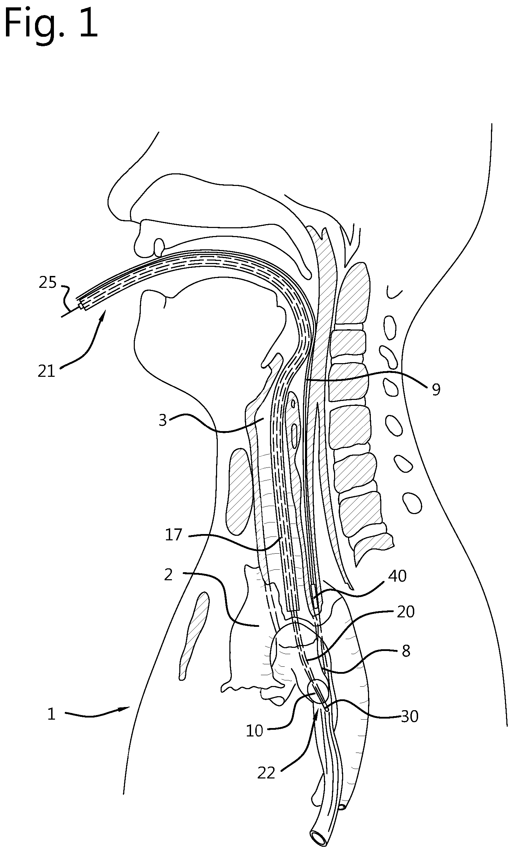

In a preferred exemplary embodiment illustrated in figure 1 the system comprises an ultrasonic imaging device 40, which enables the imaging of an organ in a patient's body 1, in particular the heart or the aorta 2, through a part of the patient's respiratory tract 3. The ultrasonic imaging device 40, for instance an echo probe, is arranged in or on the patient's body 1. In the shown embodiment, the echo probe 40, which is carried on a flexible catheter 9, is introduced into the patient's oesophagus 5. Another flexible catheter 20 carrying an inflatable member 10 is introduced into the respiratory tract 3. The inflatable member 10 is positioned at a predetermined location in the respiratory tract 3. When the organ to be imaged is the ascending aorta 2, the predetermined position will be in the top part of the left bronchus 8. The flexible catheter 20 carrying the inflatable member 10 will be guided through the patient's trachea 3 by first introducing an endotracheal tube 17 into the trachea 3. This tube 17 is somewhat stiffer than the catheter 20 and therefore easier to control. The catheter 20 is then inserted in the endotracheal tube 17. After leaving the endotracheal tube 17 the second end 22 of the catheter 20 and the inflatable member 10 are guided into the left bronchus 8.

The catheter 20 has a first end 21 intended for being located outside of the patient's body and a second end 22 intended for being located in the patient's respiratory tract. The system further comprises an imaging device 30 attached to or integrated with the second end 22 of the catheter 20. Typically the imaging device 30 comprises an image sensor and a light source, for example one or more light emitting diodes. The imaging device 30 is shaped such that the second end of the catheter 22 functions as a guide for the positioning of the inflatable member. The imaging device 30 is configured or connected to transmit image data to a device (not shown) outside the patient's body where the image data is displayed to a user. The presence of the imaging device 30 adds stiffness to the second end 22 of the flexible catheter 20, improving directional control and predictability of the movement, thereby allowing the inflatable member 10 to be swiftly and accurately positioned in the respiratory tract 3.

The catheter 20 may be made from a flexible material, preferably a polymer material, more preferably polyvinylchloride, silicone, synthetic latex. The inflatable member 10, in the non-expanded state, may have a volume between 20 ml and 60 ml, preferable between 30 ml and 50 ml. The material of the inflatable member may be a thermoplastic elastomer, preferably a thermoplastic polyurethane elastomer.

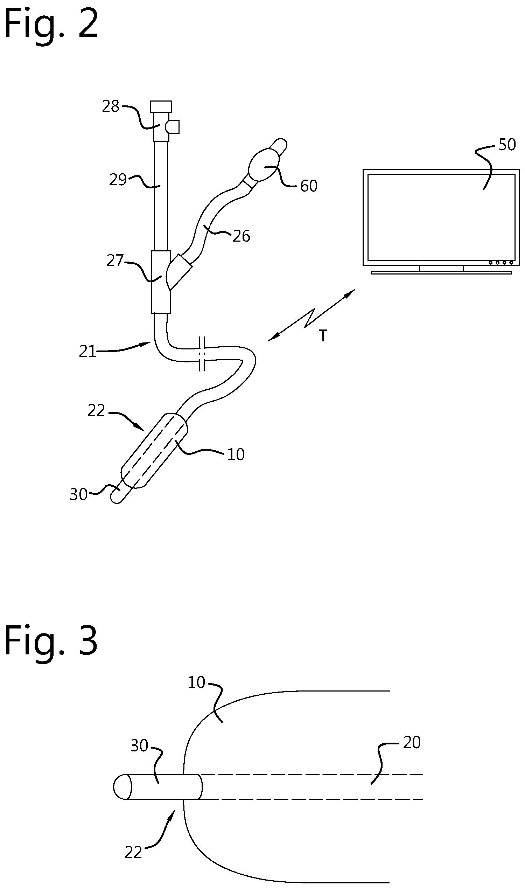

Figure 2 shows a second exemplary embodiment of a system. The system comprises at least one inflatable member 10 carried by a flexible catheter 20. For instance in the actual practice of ultrasonic imaging, the flexible catheter 20 carrying the inflatable member 10 will be guided through the patient's trachea into the right position, e.g. patient's left main bronchus. In this embodiment, the catheter comprises an imaging device 30 which is arranged at the second end 22 of the catheter 20. Positioning of the catheter 20 and the inflatable member 10 is done by manipulating the second end of the catheter 20. Figure 2 further shows a computer device 50 which is connected in a wired or wireless manner to the imaging device 30. This computer device 50 receives image data from the imaging device 30 and displays it to an operator. The imaging device 30 may have any one of the features of embodiments disclosed above and below.

The system further comprises a pressure monitoring and control member 60 to be arranged outside the patient's body, said pressure monitoring member being in fluid communication with the inflatable member 10 and being configured to receive, during operation, fluid from the inflatable member 10 when the pressure increases above a predetermined threshold pressure and to return said received fluid when the pressure decreases below said predetermined threshold pressure. The pressure monitoring and control member 60 may be an elastically expandable balloon made from a material which is configured to expand elastically when the pressure in the balloon increases above a predetermined threshold pressure and to contract when the pressure decreases below said predetermined threshold pressure.

After the inflatable member 10 has been positioned at the right position in the respiratory tract, it is filled with a fluid, e.g. water or a saline solution in minor concentrations, through the flexible catheter 20. The inflatable member 10, in the non-expanded state, has a volume between 30 ml and 70 ml, preferably between 40 ml and 60 ml. The fluid is injected into the catheter 20 by means of a syringe (not shown), which is connected to a fill connector 28 at the proximal end of a fill line 29.

The distal end of this fill line 29 in turn is connected to a proximal end of the catheter 20 through a connector 27. The degree of filling of the inflatable member 10 may be visually determined by monitoring the elastically expandable balloon 60, which is arranged at the end of a pilot line. This pilot line is also connected to the catheter 20 through the connector 27. In case of an increasing pressure within the inflatable member 10 exceeding the threshold level, e.g. during the movement of echo probe in the oesophagus 5 or the movement breathing tube in the respiratory tract, the pressure may cause a reflux of the fluid from the inflatable member 10 to the elastically expandable balloon 60. Such reflux may lead to an expansion of the elastically expandable balloon 60, thereby limiting the pressure increase within the inflatable member to a safety level. The predetermined threshold pressure of the system lies in a range between 30 and 90 mm Hg, such threshold pressure ensures a safe pressure within the inflatable member leading to no or less damage to the trachea wall.

Figure 3 shows in detail the arrangement of the imaging device 30 in the catheter 20. The imaging device is arranged at least partially in the second end 22 of the catheter. Optionally, the imaging device 30 extends partially into the zone of the inflatable member, but preferably over only a small distance (e.g. a distance which is less than 1 cm), such that the ultrasonic imaging is not disturbed.

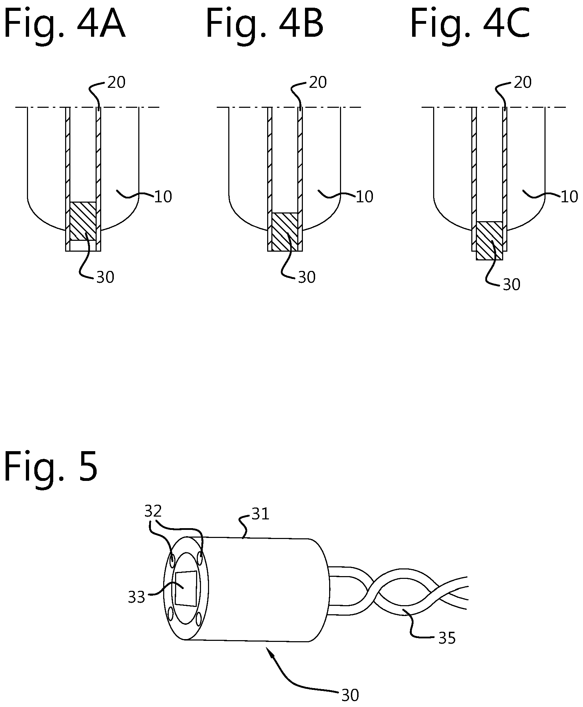

Figures 4A-4C show three possible embodiments of an imaging device 30 arranged in the second end 22 of a catheter 20. The imaging device 30 is inserted in the second end 22 of the flexible catheter 20. In the embodiment of figure 4A, the imaging device 30 is inserted entirely in the second end 22, and the flexible catheter 20 protrudes over the imaging device 30 in the longitudinal directional. In the embodiment of figure 4B, the attachment or integration is such that an end portion of the imaging device 30 is aligned with an end portion of the flexible catheter 20. Figure 4C shows an exemplary embodiment where the imaging device 30 protrudes out of the second end of the catheter 22 in the longitudinal direction.

Figure 5 shows in detail an exemplary embodiment of an imaging device. The imaging device 30 comprises a cylindrical housing 31 which is made from a material more rigid than the flexible catheter. This material could be a metal such as stainless steel and/or a plastic such as polyvinylchloride. The cylindrical housing 31 is intended to be aligned with a longitudinal axis of the catheter, as is also clear from figure 3 discussed above. Preferably, the housing 31 has a diameter between 0.01 mm and 9 mm, more preferably 0.5 mm and 5 mm, even more preferably 1 mm and 3 mm, and/or wherein a length of the housing is smaller than 10 mm, preferably smaller than 7 mm. Optionally, the housing 31 extends over a short distance in the inflatable member 10, as is also visible from figure 2 discussed above.

The imaging device 30 further comprises an image sensor 33 and at least one light source 32. The imaging device 30 may be coupled to an external power source (not shown) and may output image data through one or more electrical wires 35. Although wired solutions for both the power supply and the transferring of image data are preferred, it may also be envisaged to use a battery and/or a wireless transmission means in the imaging device 30.

The image sensor 33 is arranged in the housing 31 and may be for example an active pixel sensor such as a CMOS sensor. The light source 32 comprises a plurality of light emitting diodes arranged around the image sensor 33. The image sensor 33 may have a field of view between 90 and 180 degrees, preferably between 100 and 150 degrees.

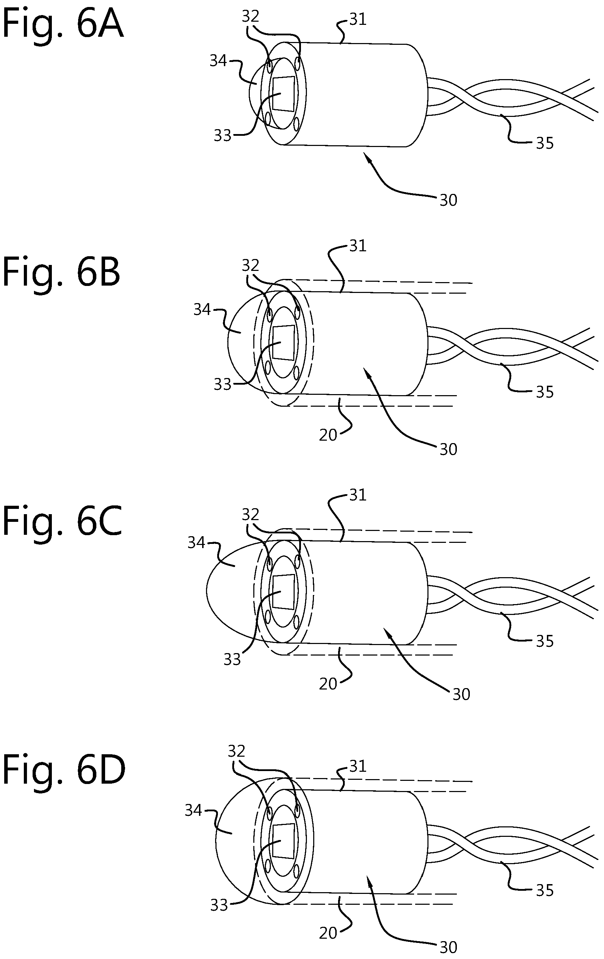

Figures 6A to 6D show exemplary embodiments of an imaging device 30 comprising a centrally positioned image sensor 33, at least one light source 32, and a lens 34 arranged in front of the image sensor 33. More in particular, figures 6A-6D show various possibilities to arrange the lens 34 at the second end of the catheter 22. In addition to its optical function, the lens 34 is used as a protection layer for the image sensor 33 and in a preferred embodiment, the stiffness and shape of the lens help to position the second end of the flexible catheter 22.

In figure 6A the lens 34 is arranged in front of the image sensor 33 and has a diameter which is smaller than the diameter of the housing 31. The diameter of the lens 34 could be bigger, smaller or equal to the diameter of the housing 31.

In figure 6B, the image sensor is surrounded by light sources 32 and the lens 34 is arranged in front of the image sensor 33 and the light sources 32 and has a diameter which is equal to the diameter of the housing 31. A skilled person knows different curvatures are possible, an exemplary embodiment of a lens 34 with a different curvature is shown in figure 6C. In another exemplary embodiment shown in figure 6D, the lens 34 is arranged in front of the image sensor 33 and has a diameter larger than the housing 31.

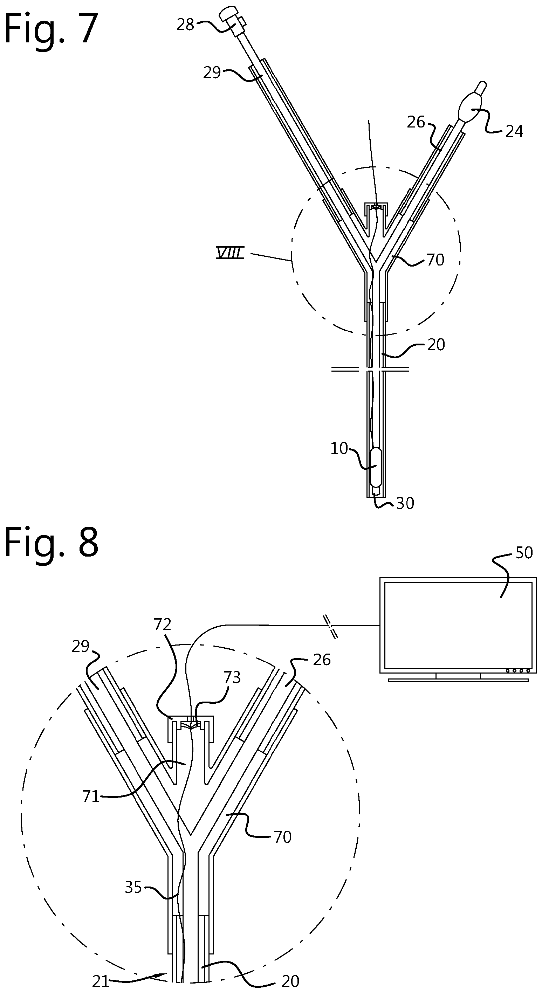

Figure 7 illustrates another exemplary embodiment of a system comprising at least one inflatable member 10 carried by a flexible catheter 20 provided with an imaging device 30 that may have any one of the features of embodiments disclosed above. Further the system comprises a fill line 29 connected to a trident connector 70 which forms the connection between the fill line 29, a pilot line 26 leading to a pilot balloon 24, and the flexible catheter 20. Figure 8 shows an enlarged view of section VIII in figure 7.

After the inflatable member 10 has been positioned, it is filled with an ultrasonic transmission fluid through the flexible catheter 20. The fluid is injected into the catheter 20 by means of a syringe (not shown) which is connected to a fill connector 20 at the end of a fill line 21 (see figure 8) . This fill line 21 in turn is connected to a proximal end of the catheter 20 through the trident connector 70. The degree of filling of the inflatable member 10 may be visually determined by monitoring the pilot balloon 24, which is arranged at the end of a pilot line 26. This pilot line 26 is also connected to the catheter 10 through the trident connector 70.

When the degree of inflation of the pilot balloon 24 indicates that the inflatable member 10 has been filled to such an extent that it completely covers the entire cross-sectional area of the left bronchus, so that no air is present between the echo probe and the organ to be imaged, the echo probe may be activated. Ultrasonic waves are then transmitted from the echo probe through the transmission fluid F in the inflatable member 10 to the ascending aorta. Reflections from the aorta are received at the echo probe and transmitted through a line running through the catheter 10 to a processing and display apparatus. Due to the presence of the inflatable member 10 that is filled with the transmission fluid F, e.g. water or a saline solution in minor concentrations, the ultrasonic waves can travel pass the respiratory tract with virtually no absorption. Consequently, very good ultrasound images of the aorta may be obtained.

The imaging device 30 may be coupled to an external power source (not shown) through one or more electrical wires 35, and the images captured by the imaging device 30 may be output through one or more external wires 35. There are various possibilities for accommodating the one or more wires 35 in the catheter 20. In a first variant, which is structurally simple, the catheter 20 has a main lumen for filling the inflatable member 10 with the ultrasonic transmission fluid and the one or more wires 35 is arranged in the main lumen. Alternatively, the catheter 20 may have a main lumen for the ultrasonic transmission fluid and an additional lumen for accommodating the one or more wires 35. In this way the one or more wires 35 does not interfere with the fluid supply function of the catheter 20. To allow the passage of the one or more wires to a device 50 outside the catheter 20, the trident connector 70 comprises a centre prong 71 and a cap 72 carrying a valve member 73.

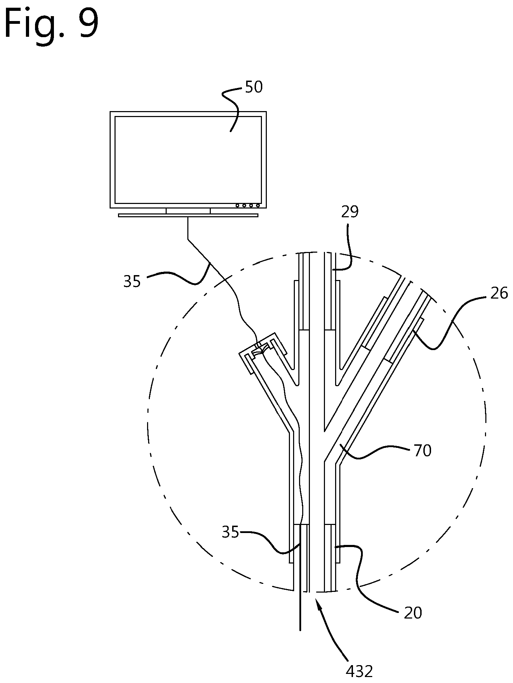

Figure 9 shows another exemplary embodiment of an alternative arrangement of the one or more electrical wires 35. This embodiment is similar to the embodiment of figures 7 and 8 with this difference that the one or more wires 35 are arranged eccentrically to the flexible catheter 20. Such an embodiment has the advantage that no special sealing is needed to bring the one or more wires 35 out of the catheter 20 as in the embodiment of figures 7 and 8. On the other hand a sealed passage may be provided at the second end to connect the one or more wires 35 to the imaging device 30.

## Claims

1. A system with an inflatable member (10) configured for being arranged in part of the patient's respiratory tract, comprising:
a catheter (20) carrying an inflatable member (10) to be arranged in the respiratory tract, said catheter being provided with a fluid line (25) for filling the inflatable member (10) with a fluid; said catheter having a first end (21) intended for being located outside of the patient's body and a second end (22) intended for being located in the patient's respiratory tract;
an imaging device (30) attached to or integrated with the second end (22) of the catheter;
wherein the imaging device (30) is configured and arranged such that the second end of the catheter (22) functions as a guide for the positioning of the inflatable member;
wherein the imaging device (30) comprises an image sensor.

2. The system of the previous claim, wherein the imaging device (30) comprises a light source (32), said light source (32) preferably comprising a plurality of light emitting diodes arranged along a periphery of the second end of the catheter (22), preferably at least three light emitting diodes equally distributed along the periphery of the second end of the catheter (22).

3. The system of any one of the previous claims, further comprising an ultrasonic imaging device (40) configured to emit ultrasound waves through the inflatable member (10) and receive reflected ultrasound waves.

4. The system of any one of the previous claims, wherein the imaging device (30) is configured or connected to transmit image data to a device (50) outside the patient's body.

5. The system of any one of the previous claims, wherein the imaging device comprises a cylindrical housing (31) aligned with a longitudinal axis of the catheter, wherein preferably the cylindrical housing (31) is arranged at least partially in the second end (22) of the catheter, preferably entirely in the second end of the catheter.

6. The system of the previous claim, wherein the housing (31) has a diameter between 0.01 mm and 9 mm, preferably 0.5 mm and 5 mm, more preferably 1 mm and 3 mm, and/or wherein a length of the housing is smaller than 10 mm, preferably smaller than 7 mm.

7. The system of any of the previous claims, wherein the imaging device (30) comprises an active pixel sensor (33), preferably a CMOS sensor.

8. The system of claim 2 and 7, wherein the plurality of light emitting diodes is arranged around the active pixel sensor (33).

9. The system of any of the previous claims, wherein the imaging device (30) comprises a lens (34) arranged in front of the active pixel sensor (33) and protruding out of the second end (22).

10. The system of any of the previous claims, wherein the imaging device (32) has a field of view between 90 and 180 degrees, preferably between 100 and 150 degrees.

11. The system of any of the previous claims, wherein the catheter (20) comprises the fluid line (25) and/or is made from a flexible material, preferably a polymer material, more preferably polyvinylchloride, silicone, synthetic latex.

12. The system of any of the previous claims, further comprising at least one electrical wire (35), preferably arranged in the catheter, and configured for connecting the imaging device with a device (50) outside the patient's body.

13. The system of any one of the previous claims, wherein the inflatable member (10), in the non-expanded state, has a volume between 20 ml and 60 ml, preferable between 30 ml and 50 ml and/or wherein the material of the inflatable member is a thermoplastic elastomer, preferably a thermoplastic polyurethane elastomer.

14. The system of any one of the previous claims, further comprising:
- a pressure monitoring and control member (60) to be arranged outside the patient's body, said pressure monitoring member being in fluid communication with the inflatable member and being configured to receive, during operation, fluid from the inflatable member (10) when the pressure increases above a predetermined threshold pressure and to return said received fluid when the pressure decreases below said predetermined threshold pressure;
- wherein the pressure monitoring and control member is an elastically expandable balloon made from a material which is configured to expand elastically when the pressure in the balloon increases above a predetermined threshold pressure and to contract when the pressure decreases below said predetermined threshold pressure.

## Patentansprüche

1. System mit einem aufblasbaren Element (10), das zum Angeordnetwerden in einem Teil der Atemwege eines Patienten konfiguriert ist, umfassend:
einen Katheter (20), der ein aufblasbares Element (10) trägt, das in den Atemwegen anzuordnen ist, wobei der Katheter mit einer Fluidleitung (25) zum Befüllen des aufblasbaren Elements (10) mit einem Fluid versehen ist; wobei der Katheter ein erstes Ende (21), das dafür vorgesehen ist, sich außerhalb des Körpers des Patienten zu befinden, und ein zweites Ende (22) aufweist, das dafür vorgesehen ist, sich in den Atemwegen des Patienten zu befinden;
eine Bildgebungsvorrichtung (30), die an dem zweiten Ende (22) des Katheters befestigt oder mit diesem integriert ist;
wobei die Bildgebungsvorrichtung (30) derart konfiguriert und angeordnet ist, dass das zweite Ende des Katheters (22) als eine Führung für die Positionierung des aufblasbaren Elements fungiert;
wobei die Bildgebungsvorrichtung (30) einen Bildsensor umfasst.

2. System des vorstehenden Anspruchs, wobei die Bildgebungsvorrichtung (30) eine Lichtquelle (32) umfasst, die Lichtquelle (32) vorzugsweise umfassend eine Vielzahl von Leuchtdioden, die entlang eines Umfangs des zweiten Endes des Katheters (22) angeordnet ist, vorzugsweise mindestens drei Leuchtdioden, die entlang des Umfangs des zweiten Endes des Katheters (22) gleichmäßig verteilt sind.

3. System nach einem der vorstehenden Ansprüche, ferner umfassend eine Ultraschallbildgebungsvorrichtung (40), die konfiguriert ist, um Ultraschallwellen über das aufblasbare Element (10) zu emittieren und reflektierte Ultraschallwellen zu empfangen.

4. System nach einem der vorstehenden Ansprüche, wobei die Bildgebungsvorrichtung (30) konfiguriert oder verbunden ist, um Bilddaten an eine Vorrichtung (50) außerhalb des Körpers des Patienten zu übertragen.

5. System nach einem der vorstehenden Ansprüche, wobei die Bildgebungsvorrichtung ein zylindrisches Gehäuse (31) umfasst, das mit einer Längsachse des Katheters ausgerichtet ist, wobei vorzugsweise das zylindrische Gehäuse (31) mindestens teilweise in dem zweiten Ende (22) des Katheters, vorzugsweise vollständig in dem zweiten Ende des Katheters angeordnet ist.

6. System des vorstehenden Anspruchs, wobei das Gehäuse (31) einen Durchmesser zwischen 0,01 mm und 9 mm, vorzugsweise 0,5 mm und 5 mm, mehr bevorzugt 1 mm und 3 mm aufweist, und/oder wobei eine Länge des Gehäuses kleiner als 10 mm, vorzugsweise kleiner als 7 mm ist.

7. System nach einem der vorstehenden Ansprüche, wobei die Bildgebungsvorrichtung (30) einen aktiven Pixelsensor (33), vorzugsweise einen CMOS-Sensor umfasst.

8. System nach Anspruch 2 und 7, wobei die Vielzahl von Leuchtdioden um den aktiven Pixelsensor (33) herum angeordnet ist.

9. System nach einem der vorstehenden Ansprüche, wobei die Bildgebungsvorrichtung (30) eine Linse (34) umfasst, die vor dem aktiven Pixelsensor (33) angeordnet ist und aus dem zweiten Ende (22) herausragt.

10. System nach einem der vorstehenden Ansprüche, wobei die Bildgebungsvorrichtung (32) ein Sichtfeld zwischen 90 und 180 Grad, vorzugsweise zwischen 100 und 150 Grad aufweist.

11. System nach einem der vorstehenden Ansprüche, wobei der Katheter (20) die Fluidleitung (25) umfasst und/oder aus einem flexiblen Material, vorzugsweise einem Polymermaterial, mehr bevorzugt Polyvinylchlorid, Silikon, synthetischem Latex hergestellt ist.

12. System nach einem der vorstehenden Ansprüche, ferner umfassend mindestens einen elektrischen Draht (35), der vorzugsweise in dem Katheter angeordnet und zum Verbinden der Bildgebungsvorrichtung mit einer Vorrichtung (50) außerhalb des Körpers des Patienten konfiguriert ist.

13. System nach einem der vorstehenden Ansprüche, wobei das aufblasbare Element (10), in dem nicht ausgedehnten Zustand, ein Volumen zwischen 20 ml und 60 ml, vorzugsweise zwischen 30 ml und 50 ml aufweist und/oder wobei das Material des aufblasbaren Elements ein thermoplastisches Elastomer, vorzugsweise ein thermoplastisches Polyurethanelastomer ist.

14. System nach einem der vorstehenden Ansprüche, ferner umfassend:
- ein Drucküberwachungs- und Steuerelement (60), das außerhalb des Körpers des Patienten anzuordnen ist, wobei das Drucküberwachungselement in Fluidkommunikation mit dem aufblasbaren Element steht und konfiguriert ist, um, während eines Betriebs, Fluid von dem aufblasbaren Element (10) zu empfangen, wenn der Druck über einen vorbestimmten Schwellendruck ansteigt, und um das empfangene Fluid zurückzuführen, wenn der Druck unter den vorbestimmten Schwellendruck abfällt;
- wobei das Drucküberwachungs- und Steuerelement ein elastisch ausdehnbarer Ballon ist, der aus einem Material hergestellt ist, das konfiguriert ist, um sich elastisch auszudehnen, wenn der Druck in dem Ballon über einen vorbestimmten Schwellendruck ansteigt, und um sich zusammenzuziehen, wenn der Druck unter den vorbestimmten Schwellendruck abfällt.

## Revendications

1. Système avec un élément gonflable (10) conçu pour être agencé dans une partie des voies respiratoires du patient, comprenant :
un cathéter (20) portant un élément gonflable (10) afin d'être agencé dans les voies respiratoires, ledit cathéter étant pourvu d'une ligne de fluide (25) pour remplir l'élément gonflable (10) avec un fluide ; ledit cathéter ayant une première extrémité (21) destinée à être située à l'extérieur du corps du patient et une seconde extrémité (22) destinée à être située dans les voies respiratoires du patient ;
un dispositif d'imagerie (30) fixé à ou intégré à la seconde extrémité (22) du cathéter ;
dans lequel le dispositif d'imagerie (30) est configuré et agencé de telle sorte que la seconde extrémité du cathéter (22) fonctionne comme un guide pour le positionnement de l'élément gonflable ;
dans lequel le dispositif d'imagerie (30) comprend un capteur d'image.

2. Système selon la revendication précédente, dans lequel le dispositif d'imagerie (30) comprend une source de lumière (32), ladite source de lumière (32) comprenant de préférence une pluralité de diodes électroluminescentes agencées le long d'une périphérie de la seconde extrémité du cathéter (22), de préférence au moins trois diodes électroluminescentes également réparties le long de la périphérie de la seconde extrémité du cathéter (22).

3. Système selon l'une quelconque des revendications précédentes, comprenant en outre un dispositif d'imagerie à ultrasons (40) configuré pour émettre des ondes ultrasonores à travers l'élément gonflable (10) et recevoir des ondes ultrasonores réfléchies.

4. Système selon l'une quelconque des revendications précédentes, dans lequel le dispositif d'imagerie (30) est configuré ou connecté pour transmettre des données d'image à un dispositif (50) extérieur au corps du patient.

5. Système selon l'une quelconque des revendications précédentes, dans lequel le dispositif d'imagerie comprend un boîtier cylindrique (31) aligné sur un axe longitudinal du cathéter, dans lequel, de préférence, le boîtier cylindrique (31) est agencé au moins partiellement dans la seconde extrémité (22) du cathéter, de préférence entièrement dans la seconde extrémité du cathéter.

6. Système selon la revendication précédente, dans lequel le boîtier (31) a un diamètre compris entre 0,01 mm et 9 mm, de préférence 0,5 mm et 5 mm, plus préférablement 1 mm et 3 mm, et/ou dans lequel une longueur du boîtier est inférieure à 10 mm, de préférence inférieure à 7 mm.

7. Système selon l'une quelconque des revendications précédentes, dans lequel le dispositif d'imagerie (30) comprend un capteur de pixel actif (33), de préférence un capteur CMOS.

8. Système selon la revendication 2 et 7, dans lequel la pluralité de diodes électroluminescentes est agencée autour du capteur de pixel actif (33).

9. Système selon l'une quelconque des revendications précédentes, dans lequel le dispositif d'imagerie (30) comprend une lentille (34) agencée devant le capteur de pixel actif (33) et faisant saillie hors de la seconde extrémité (22).

10. Système selon l'une quelconque des revendications précédentes, dans lequel le dispositif d'imagerie (32) a un champ de vision compris entre 90 et 180 degrés, de préférence entre 100 et 150 degrés.

11. Système selon l'une quelconque des revendications précédentes, dans lequel le cathéter (20) comprend la ligne de fluide (25) et/ou est constitué d'un matériau flexible, de préférence un matériau polymère, plus préférablement polychlorure de vinyle, silicone, latex synthétique.

12. Système selon l'une quelconque des revendications précédentes, comprenant en outre au moins un fil électrique (35), de préférence agencé dans le cathéter, et configuré pour connecter le dispositif d'imagerie à un dispositif (50) extérieur au corps du patient.

13. Système selon l'une quelconque des revendications précédentes, dans lequel l'élément gonflable (10), à l'état non déployé, a un volume compris entre 20 ml et 60 ml, de préférence entre 30 ml et 50 ml et/ou dans lequel le matériau de l'élément gonflable est un élastomère thermoplastique, de préférence un élastomère de polyuréthane thermoplastique.

14. Système selon l'une quelconque des revendications précédentes, comprenant en outre :
- un élément de surveillance et de commande de pression (60) à agencer à l'extérieur du corps du patient, ledit élément de surveillance de pression étant en communication fluidique avec l'élément gonflable et étant conçu pour recevoir, pendant le fonctionnement, un fluide de l'élément gonflable (10) lorsque la pression augmente au-dessus d'une pression seuil prédéterminée et pour renvoyer ledit fluide reçu lorsque la pression diminue en dessous de ladite pression seuil prédéterminée ;
- dans lequel l'élément de surveillance et de commande de pression est un ballonnet élastiquement déployable fabriqué à partir d'un matériau conçu pour se déployer élastiquement lorsque la pression dans le ballonnet augmente au-dessus d'une pression seuil prédéterminée et pour se contracter lorsque la pression diminue en dessous de ladite pression seuil prédéterminée.
